## Europäisches Patentamt
## European Patent Office
## Office européen des brevets

(19)

(11) Publication number: **0 005 864**
**B1**

(12) **EUROPEAN PATENT SPECIFICATION**

(45) Date of publication of patent specification: **21.10.81**

(51) Int. Cl.³: **A 61 K 39/29, C 12 N 7/00**

(21) Application number: **79101777.5**

(22) Date of filing: **06.06.79**

(54) Antigenic composition useful as a vaccine or vaccine intermediate, and process for preparing same.

(30) Priority: **07.06.78 US 913422**

(43) Date of publication of application:
**12.12.79 Bulletin 79/25**

(45) Publication of the grant of the European patent:
**21.10.81 Bulletin 81/42**

(84) Designated Contracting States:
**AT BE DE FR GB NL SE**

(56) References cited:
**FR - A - 2 331 354**

(73) Proprietor: **New York Blood Center, Inc.**
**310 East 67 Street**
**New York, New York 10021 (US)**

(72) Inventor: **Prince, Alfred Mayer**
**Stone Hill Road Pound Ridge**
**New York 10576 (US)**
Inventor: **Vnek, John Andrew**
**5133 Post Road Bronx**
**New York 10471 (US)**

(74) Representative: **Splanemann, Rainer et al,**
**Patentanwälte R. Splanemann Dr. B. Reitzner, J.**
**Richter F. Werdermann Tal 13**
**D-8000 München 2 (DE)**

Courier Press, Leamington Spa, England.

Antigenic composition useful as a vaccine or vaccine intermediate, and process for preparing same

This invention relates to an antigenic composition useful as a vaccine or vaccine intermediate. More especially, this invention relates to an antigenic composition containing subviral particles which contain hepatitis B surface and/or HBe antigens. This invention is directed to both the antigenic composition and a method of making the same.

Recently, considerable attention has been directed to preparing a concentrated antigenic mass containing particles of hepatitis B surface antigen (HBsAg). A number of approaches have been considered. For instance, Blumberg, in U.S. 3,636,191, disclosed the preparation of a hepatitis B surface antigen-containing vaccine by subjecting suitable sera containing the antigen to a series of enzyme digestion steps. As a result of these sequential enzyme digestions, a concentrated HBsAg-containing composition was said to be produced. The composition following purification contained hepatitis B surface antigen on particles having a particle size of about 20 nm. Blumberg recognized that the 20 nm hepatitis B surface antigen associated particles were in the form of a shell. Blumberg's composition had no core and was substantially resistant to various enzymes employed in the digestion procedure. Blumberg provided a vaccine having a density on the order of 1.21 g/cc which could be diluted with a physiologically acceptable medium and employed as an active immunizing agent.

An improvement in the method of preparing an HBsAg containing vaccine was disclosed by Prince et al in U.S. 3,951,937, the disclosure of which is hereby incorporated herein specifically by reference, which process involves the use of a series of treatments of HBsAg positive sera with polyethylene glycol whereby, after several physical separation procedures, a concentrated HBsAg mass was provided wherein the viral components could be readily inactivated, (i.e., non-infectious), and the same employed as a vaccine. The process has been considered to be useful in the large scale purification of HBsAg.

Other approaches have been followed to prepare vaccines which elicit an active immunological response. Thus, in United States Patent No. 4,118,479 entitled VACCINE FOR ACTIVE IMMUNIZATION CONTAINING HEPATITIS B SURFACE ANTIGEN AND ASSOCIATED ANTIGEN, the disclosure of which is hereby incorporated herein specifically by reference, there is described a process for preparing a vaccine against viral hepatitis which contains particles in the range of 30 to 50 nanometers. Also disclosed in that application is such a vaccine which contains a hepatitis B antigenic specificity which has been termed "e-antigen" (HBeAg).

Thus, it has heretofore been recognized that vaccines against viral hepatitis were to be characterized by particles of a size of at least 20 nanometers. Moreover, it has been recognized that especially active vaccines, characterized by HBsAg and e-antigen, could be prepared only from those plasmas having HBsAg, e-antigen, and particles in the range of 30 to 50 nanometers. It was recognized, of course, that optimally a vaccine for use in humans would have to be safe and such vaccine, therefore, would have to be one wherein the viral components of the concentrated mass were inactivated. Inactivation of the infection virion, according to the above-mentioned United States Patent can be performed by serial treatment with cobalt irradiation, formalin, and beta-propiolactone, using conventional procedures.

It became desirable to provide a safe vaccine against viral hepatitis (Type B) containing HBsAg and HBeAg but lacking potentially dangerous components of the Dane particle core, such as HB virus DNA and DNA polymerase. As it has been learned that HBe antigen is a useful component of the vaccine, providing additional protection against reinfection, and as HBeAg is preferentially associated with larger particles, such as the HB virion (the Dane particle), the difficult task was posed of preparing a vaccine out of plasma containing a high content of virions, and thus potentially dangerous, isolating the protective surface components (HBsAg and HBeAg), and removing from the final product the core components (HB DNA polymerase).

In accordance with the present invention, it has been found that an active vaccine against viral hepatitis can be provided by subjecting human or other animal plasma to purification procedures to isolate particles containing HBsAg, preferably including a high content of Dane particles, followed by an inactivation step which comprises solubilizing the HBsAg particles, where the particles have a size of at least 16 nanometers, with a detergent. According to the process, an ionic or nonionic detergent is employed which disperses at the prevailing temperature up to 0.1 percent by weight fat in an aqueous solution containing 0.1 percent by weight fat when 1 gram/100 ml of detergent is introduced therein. Suitable detergents include polyoxyethylene derivatives of fatty acids partial esters of sorbitol anhydrides (for example, commercial products such as Tween 80 and Tween 20), which are polyoxyethylene derivatives of sorbitan monooleate and monolaurate, respectively nonionic, cationic and anionic types; oil-soluble and water soluble types (e.g., commercially available Triton X 100), sodium deoxycholate, "Zwittergents", i.e., synthetic zwitterionic detergents known as sulfobetaines such as N-dodecyl-N,N-dimethyl-2-ammonia-1-ethane sulfonate and its congeners, or nonionic detergents such as Octyl-B-D-glucopyranoside. The detergent is added to the antigenic mass containing HBsAg particles of at least 16 nanometers diameter. The mixture is held under conditions sufficient to produce total dissolution of its particles. These conditions will vary with detergent concentration, HBsAg concentration, time, temperature, ionic strength and pH. Generally, the detergent is employed in the form of an aqueous solution. Its concentration in such aqueous solution will be between 0.1 and 2.0 weight percent, preferably between 0.5 and 1.0 weight percent. At 37°C, an HBsAg concentration of 0.5 mg/ml, and a neutral buffer containing 0.01 to 0.1 M salt, a detergent exposure of 1—10 days, preferably 2—4 days, is required. Higher salt concentrations reduce time and temperature requirements.

As a result of such treatment, there is provided an antigenic composition, useful as a vaccine or vaccine intermediate, which comprises a subparticulate mass, which mass contains hepatitis B surface and HBe antigens. Stated differently, as a result of the detergent treatment, the particles are fragmented, whereby an antigenic mass having subparticulates results where the subparticulates each have a size less than 20 nanometers, especially less than 16 nanometers, and more especially less than 5 nanometers.

It was surprising to find that even mild nonionic detergents such as Tween 80 will disrupt the 20 nm particle if held together with these particles at 37°C for a sufficient period of time. The previous literature and experience had suggested that Tween 80, and similar nonionic detergents, do not disrupt these particles; however these experiments had not been carried out at a sufficiently high temperature, or for a sufficient duration, to effect the solubilization which has now been observed. Strong ionic detergents, e.g., sodium dodecylsulfate, will solubilize the 20 nm particles; however this results in unacceptable denaturation of the constituent proteins with substantial resultant loss of antigenicity and immunogenicity. What is also considered to be particularly surprising is that notwithstanding the fragmentation of the HBsAg-bearing particles, the antigenic mass retains essentially all HBsAg and HBeAg activity. Thus, there is provided a vaccine composition which is safe, in that it is free of potentially harmful components of the hepatitis virus and yet is highly immunogenic. The antigenic composition of the invention is further characterized by the presence of lipids intimately associated with the protein moiety, giving the resultant antigen a density between 1.21 and 1.24 g/cc at 8°C in CsCl.

The subparticulate antigenic composition of the invention is characterized by these subparticles which bear HBsAg and HBeAg. The subparticles, according to the invention, desirably have an especially small particle size, preferably less than 5 nanometers, and preferably are in the range of between 1 and 4 nanometers. As a result of the detergent treatment of the large particles, followed by subsequent purification, the antigenic composition is free of detectable quantities of deoxyribonucleic acid (DNA), DNA polymerase, and hepatitis B core antigen (HBcAg).

The HBsAg antigenic composition which is subjected to detergent treatment can be of widely varying nature. It can be a finished vaccine which has been subjected to previous inactivation procedures or it can be any intermediate up to that stage of purification. It can be a composition provided by subjecting HBsAg positive plasma to a series of enzymatic digestions or it can be HBsAg obtained by removal of normal human serum proteins by physical separation procedures, including the aforedescribed polyethylene glycol treatment. Thus, one can subject the Blumberg vaccine composition, characterized by particles of a size of 22 nm, to the aforedescribed detergent treatment to thereby obtain a composition at least as safe, and probably safer, than the vaccine described by Blumberg which contains the HBsAg on particles of substantially greater size, and could thus contain residual traces of infective viral particles.

Alternatively, one can employ as the starting material an impure HBsAg-containing mass, such as is provided by some, but not all, of the enzyme digestions of Blumberg. Moreover, the procedure is useful in the treatment of those candidate HBsAg vaccine known as the "Purcell/ Gerin" (N.I.H.) vaccine; the Merck Sharpe Dohme vaccine; the MAUPAS or Institute Pasteur vaccines; as well as the vaccine developed by the New York Blood Center, Inc. Detergent treatment is equally applicable to the treatment of e-antigen (HBeAg) positive sera or negative HBsAg compositions, although it is especially useful in the treatment of HBsAg containing large particles of the size of 30 to 44 nanometers, i.e., Dane particles and filaments.

In carrying out the process, it is preferred to subject HBsAg to contact with detergent in accordance with the following steps:

A. Contacting the antigenic mass containing particles of the size of at least 20 nanometers initially with detergent for a period of time of between 1 and 4 hours and

B. Thereafter contacting the detergent-containing antigenic composition with an aqueous aldehyde solution. Preferably, the aldehyde is formaldehyde introduced in the form of an aqueous solution where the formaldehyde is present in a concentration of between 35 and 40 weight percent, especially 37 percent (formalin). The amount of formalin is between one part per thousand and one part per 4,000 (v/v).

The aldehyde treatment is performed by maintaining the detergent-containing antigenic composition in contact with the aldehyde at a temperature between 4 and 90°C preferably between 7 and 37°C for between 1 and 10 days. Preferably, between the initial contact with the detergent and the treatment of the detergent-containing antigenic composition with the aldehyde, the mass is subjected to sterile filtration. It is furthermore desirable to subject the aldehyde treated product (following contact at 4 to 90°C for between 1 and 10 days) to diafiltration. When the diafiltration has been effected, the

3

resultant mass can be diluted to the desired HBsAg concentration, suitably between 10 and 100 $\mu$gm/ml. The mass can be buffered to a pH of 7.0 $\pm$ 0.5. The final vaccine is then subjected to sterile filtration, filling and lyophilization.

It is preferred, for preparation of a highly active HBsAg vaccine, that the antigenic mass subjected to the detergent treatment contain HBsAg particles with diameters in the range of 16 to 50 nanometers, and that these particles contain e-antigen. Similarly, it is preferred that at least some of these particles be Dane particles and filaments. As a result of the treatment, subparticulate HBsAg and HBeAg containing antigenic mass is provided which is free of: Dane particles; filaments; 20 nanometer particles; detectable DNA; DNA polymerase; and hepatitis B core antigen. It is likely that the absence of core particles, DNA, DNA polymerase and HBcAg is due to a complexing of isolated core particles with anti-HBc which is present within some of the Dane particles and is released into solution by the action of the detergent. The resultant immune complexes (core particles + anti-HBc) cannot pass through the final sterilization filters (millipore 0.45 and 0.22 micron average pore diameter), and are thus completely removed from the final vaccine. HBeAg antigen is still present as determined by radioimmunoassay. Following the entire inactivation process, approximately 50 percent of the HBsAg antigenicity, relative to the starting plasma, has been retained. The final product is conveniently stabilized with 0.5 mg/ml of human serum albumin. The vaccine is clearly subparticulate. It is substantially free of anti-HBs and anti-HBc antibodies. The subviral antigenic particles have a density of between 1.20 and 1.24 grams per cc, determined by CsCl isopycnic banding at 8°C, and molecular weights (Daltons) in the range of 20,000 to 1,000,000, especially 200,000 to 500,000. The process can be performed to provide the HBsAg in a wide variety of concentrations. Preferably, the concentration of such HBsAg-bearing particles is at least 0.001 percent by weight, preferably between 0.001 and 0.01 weight percent. Thus, HBsAg is present in an amount sufficient to effect an anti-HBs response when administered to an animal or to man.

The process is especially effective and fragments virtually all of the HBsAg-bearing particles of a size of 20 nanometers or more, so that the resultant composition is substantially free of particles of a 16 nanometer or larger size.

Referring to the photographs appended hereto:

Fig. 1 is a series of electron microscopes of purified HBsAg. Pictures were taken after negative staining with phosphotungstate in a JEOL 100B electron microscope at 80,000X magnification.

    a.    Rate zonal centrifugation — Pool I: Dane particles and large filaments.

    b.    Rate zonal centrifugation — Pool II: filaments and 22—28 nm spherical particles.

    c.    Rate zonal centrifugation — Pool III: 20—28 nm spherical particles;

Figure 2 is a similar electron microscope of the product of the invention.

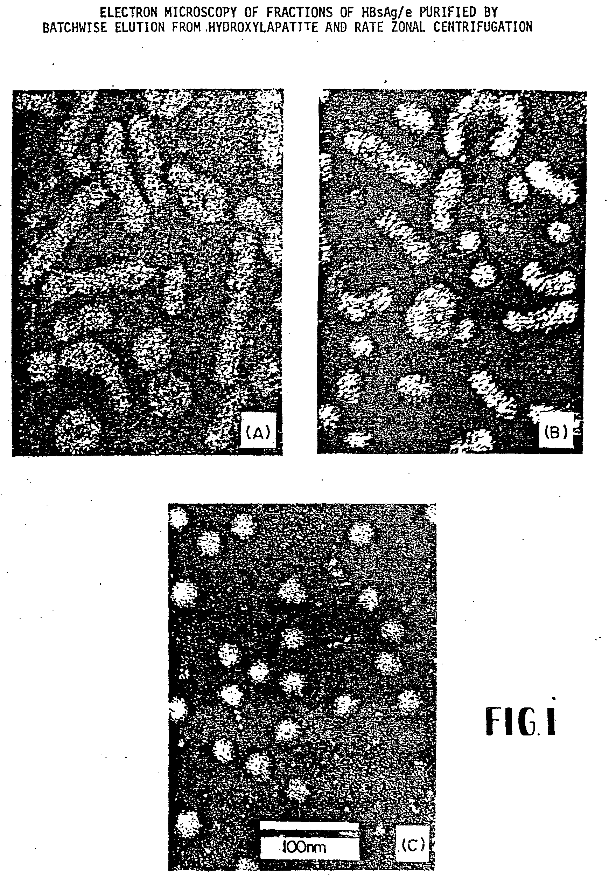

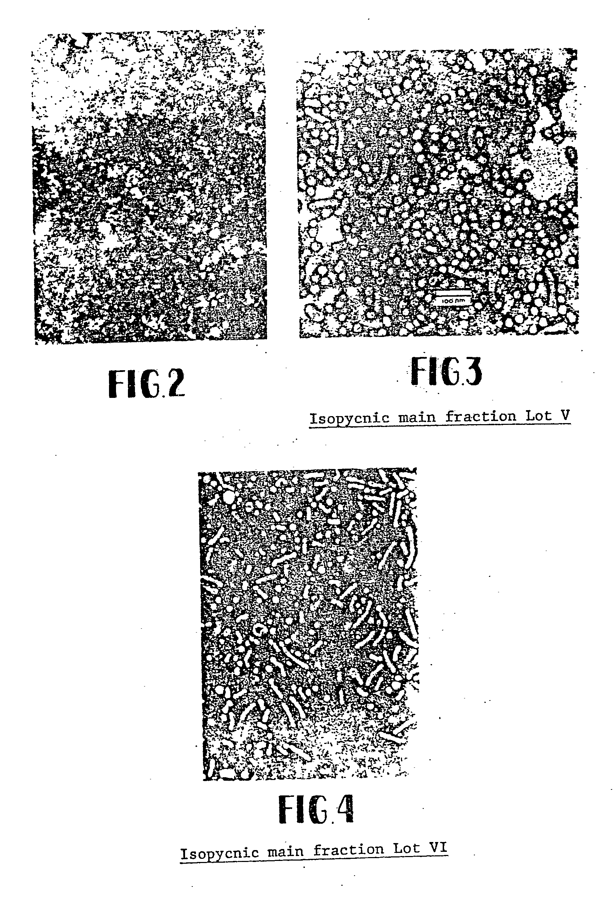

From a comparison of Figure 1 with Figure 2, it is clear that the morphology of the subparticulate HBsAg mass of the invention is decidedly unlike the particles of HBsAg in vaccines heretofore provided.

Figures 3 and 4 are similar electron microscopes of intermediates before solubilization by treatment in accordance with the invention.

Referring to the graphs and diagrams herein:

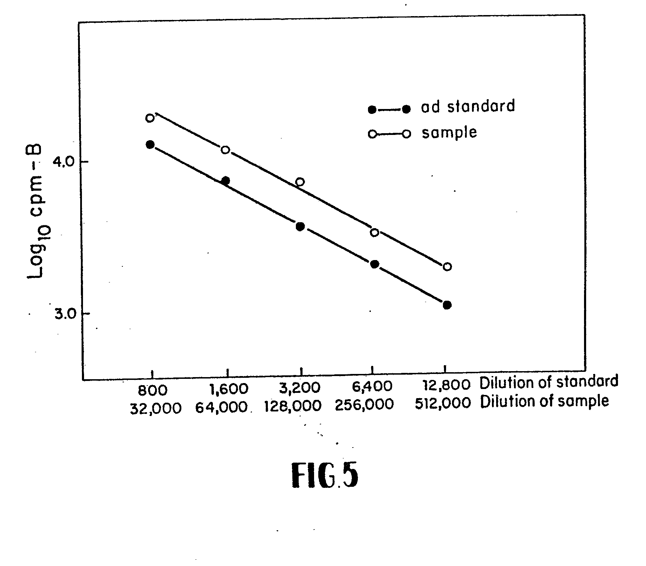

Figure 5 is a graph plotting the log dilution and log CPM-B indicating the method which has been used for quantitation of HBsAg in the product of the invention.

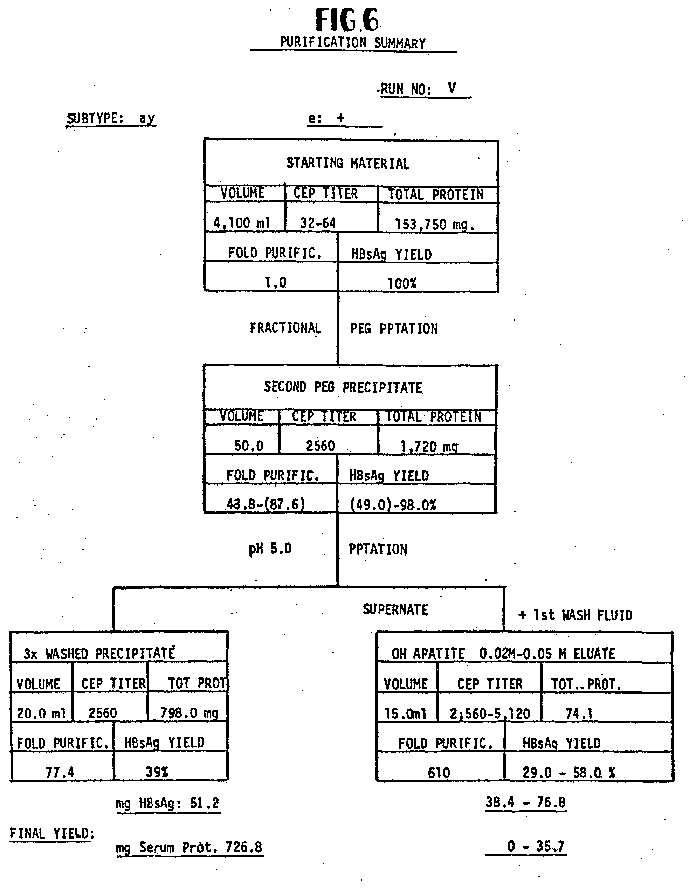

Figure 6 is a flow diagram of a process for carrying out the invention (Lot V).

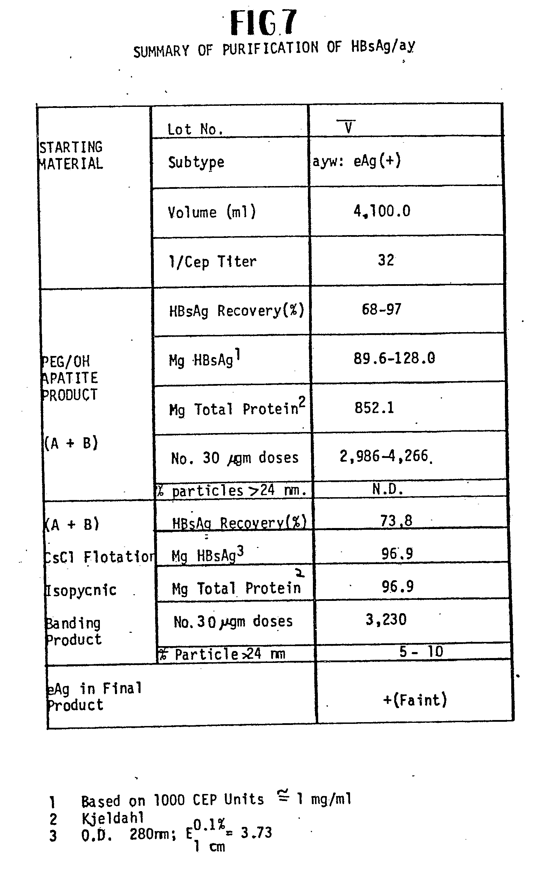

Figure 7 is a summary of purification data obtained by carrying out the invention (Lot V).

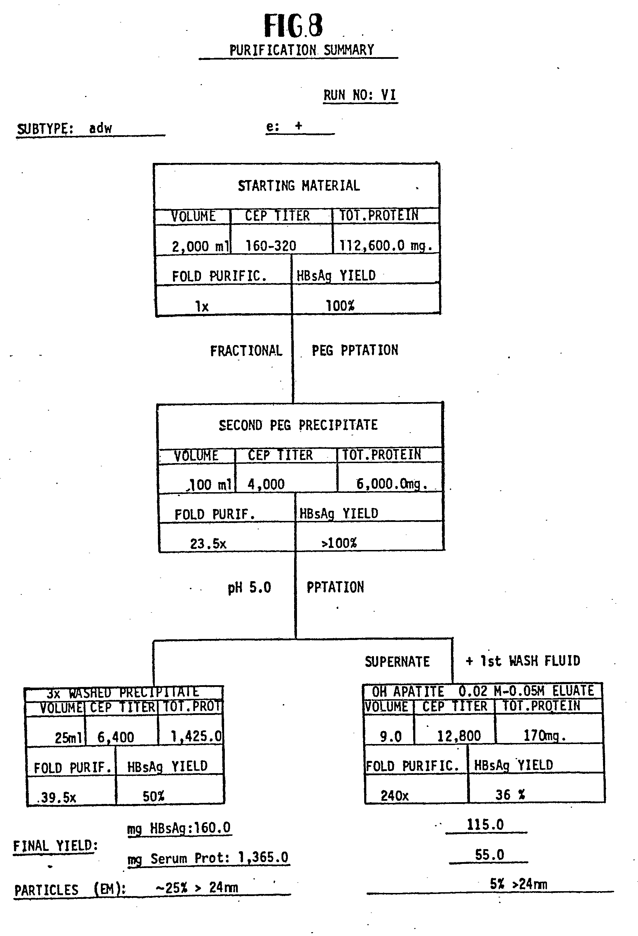

Figure 8 is another flow diagram of a process for carrying out the invention (Lot VI).

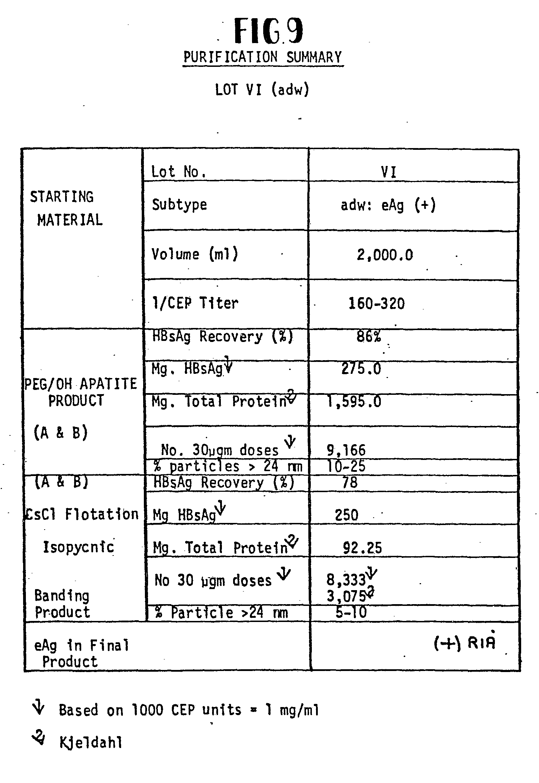

Figure 9 is a table similar to Figure 7, i.e., a summary of purification data obtained by carrying out the invention (Lot VI).

Preferably, one commences the preparation of a vaccine or vaccine intermediate of the invention by selection of HBsAg positive plasma. Preferably, the plasma is one which contains e-antigen. Only 1 to 10 percent of chronic HBsAg carriers found among blood donors satisfy this criterion. The plasma should be free of anti-e anti-body. Preferably, the plasma contains Dane particles detectable by conventional techniques of electron microscopy (negative staining); of course the plasma also contains the BhsAg-bearing particles. The starting material is initially frozen at −70°C within four hours of collection and held at 4°C during the interval prior to freezing. It should be bacteriologically sterile.

The plasma containing HBsAg is then purified to remove impurities, notably normal human serum proteins, by initially maintaining the pH of the blood plasma within the range of approximately 4.4 to 4.7, resulting in a small precipitate which, together with remaining cells and cell debris, can be removed by moderate speed centrifugation, e.g., 20 minutes at 10,000 rpm. Thereafter, the material is mixed with 2.5 to 4.5 weight percent of polyethylene glycol (PEG), preferably 3.0 to 4.5. The lower concentrations, say 3—4 percent, can selectively precipitate large particle forms and, therefore, are advantageous under certain circumstances. The pH is maintained at 4.4 to 4.7. The weight percent of polyethylene glycol is based upon the total weight of the mixture. This precipitates hepatitis B-containing antigen material of large particle size, e.g., 30 to 50 nanometers, as well as a proportion of the known shell-like hepatitis B surface antigen-containing material. The precipitate is recovered and an amount of water is added such as to present an intermediate fluid having an antigen concentration the same or higher as in the original blood material. The pH is intermediate fluid is suggested in the range

**0 005 864**

of about 4.9 to 5.1, optimally 5.0, whereby there is formed a precipitate containing proteinaceous material and polyethylene glycol and a fluid phase containing type B hepatitis antigen further characterized by the presence of Dane particles and/or filamentous material. The fluid phase is separately recovered and the pH adjusted to be in the range of 4.4 to 4.7. The fluid phase is thereafter admixed while it is maintained in this pH range with 3.0 to 4.5 weight percent polyethylene glycol, based upon the total weight of the mixture.

Preferably, in such a separation, the temperature of each of the mixtures after the mixing steps is maintained in the range of 0 to 8°C during the production of the precipitates. The purification step is facilitated if recovery is augmented by the use of centrifugation following the precipitation steps. Polyethylene glycol which is employed can be used as such, or in the form of an aqueous solution, preferably one which is about 30 percent by weight polyethylene glycol. Generally, this polyethylene glycol is one which has a molecular weight in the range of 500 to 50,000, preferably about 6,000.

Further purification can be effected by subjecting the purified antigens to absorption of hydroxylapatite using batch or column chromatographic procedures. In the latter instance, the partially purified antigens are passed through a chromatographic column containing hydroxylapatite to which they adsorb. Further purified antigens are then recovered using a multi-, e.g., about 3-step, elution procedure. Particles of particle size 25 to 50 nm are recovered separately from a fraction of particles of smaller size (16—22 nm) and the bulk of serum proteins. Additional purification can then be achieved by flotation isopycnic banding in a suitable density gradient as described elsewhere (Prince, A.M., Vnek, J., "Comparative Evaluation of Hepatitis B Vaccines" in *Symposium on Viral Hepatitis,* University of California, San Francisco, Franklin Institute Press, 1978, in press), the disclosure of which is hereby incorporated specifically herein by reference, followed by the inactivation steps described above.

The composition provided by the process of the invention can be used as such as an active vaccine for viral hepatitis. The vaccine's concentration is adjusted to about 5 to 100 $\mu$g, preferably 20 to 50 $\mu$gm, of HBsAg associated protein per dose, to give suitable immunogenicity. Antigenicity is determined by quantitative radioimmunoassay by comparison with the Bureau of Biologics, F.D.A., Vaccine Standard preparation.

Potency is also controlled by determining the minimal concentration of each batch of vaccine which gives a definite specific anti-HBs response in guinea pigs, mice, or other suitable test animals. The vaccine must have a sufficient potency to provide an anti-HBs titer of at least 50 milli-International Units (M.I.U.) of anti-HBs (as determined by quantitative radioimmunoassay by comparison with the WHO International Standard Hepatitis B immune globulin preparation) with a vaccine dose of 50 micrograms HBsAg given in two 50 microgram doses one month apart. Potency can be determined in test animals such as chimpanzees. Furthermore, anti-HBs must remain detectable at a titer of greater than 10 M.I.U. for at least one year following the onset of immunization of these chimpanzees. Preferably, a vaccine is employed containing e-antigen, in which case the chimpanzees desirably have an anti-e response measurable by radio-immunoassay.

The vaccine can be administered by sub-cutaneous or intramuscular injection. While the optimum route has not been determined, it is preferred to administer the vaccine by intramuscular injection. The frequency of administration is usually about two doses one month apart, which may be followed by a booster at six months to one year after primary immunization. Of course, the dosage depends upon the size of the host animal being inoculated. The subsequent doses or booster depends upon the level of antibody in the blood as a result of the initial immunization. Licensable adjuvants conventionally employed in vaccine manufacture can be utilized.

The vaccine is recommended for all persons at risk of developing hepatitis B infection, and particularly those at especially high risk, such as patients and staff on hemodialysis units, medical personnel, persons of tropical populations and those visiting the tropics. In the case of tropical populations, particularly in Africa, Asia, the Mediterranean region and South America, where a high incidence of hepatitis B infections has been consistently observed, the vaccine should be administered sufficiently early in life to prevent acquisition of chronic carrier state infections which tend to occur in these regions during the first five years of life.

When adequate supplies are available and effectiveness has been documented, the vaccine will be useful for all persons not already protected against hepatitis B infections as a result of prior immunity.

The particular importance of hepatitis B vaccine lies in its role in prevention of the chronic hepatitis B carrier state with its attendant risk of development of chronic liver diseases, such as chronic acitve hepatitis, cirrhosis and hepatoma. The association of a chronic hepatitis B carrier state and these diseases is now abundantly documented, and the role of chronic hepatits B as a cofactor in the etiology of these conditions is also established [Prince, A. M. in *The Liver: Normal and Abnormal Functions,* edited by F. F. Becker (M. Dekker, New York) 1975].

The use of the new hepatitis B-containing vaccine in the long run can have a significant effect in the prevention of these chronic liver diseases which are so extraordinarily prevalent in those regions, primarily in the tropics, where chronic hepatitis B carrier state is usually frequent. For example, in Senegal, where 14 percent of the population chronically carry HBsAg, the attack rate of primary liver cancer is 50 to 100 times as high as that observed in the United States, where only an average of

5

approximately 0.3 percent of the population are chronic carriers of hepatitis B virus.

In order to more fully illustrate the nature of the invention and the manner of practicing the same, the following examples are presented:

Examples
Large Scale Purification of HBsAg

### A. PEG precipitation

1.) *Pre-purification:* The pooled plasma is adjusted to pH 4.6 and 40 percent PEG (in distilled $H_2O$) is added to a final concentration of 2 percent (w/v). The sample is centrifuged in a Sorvall RG—3 centrifuge at 3,000 rpm for 20 minutes, and the sediment is discarded. The same centrifugation conditions are used throughout the PEG prodecure.

2.) *First PEG precipitation of HBsAg:* The PEG concentration in tne supernatant from the previous step is raised to a 4 percent final concentration. The solution is chilled on ice for 2 hours and HBsAg is removed with the precipitate by centrifugation.

3.) *Removal of PEG:* The precipitate is resuspended in distilled water to 1/4 of the original volume. The bulk of PEG and some accompanying proteins are removed by centrifugation after adjusting the pH to 5.0 and chilling the sample on ice for 1 to 2 hours.

4.) *Second PEG precipitation of HBsAg:* The HBsAg from the previous step of purification (pH 5.0 supernatant) is precipitated by re-adjusting to pH 4.6 and further addition of PEG to a final concentration of 3 percent. The preparation is chilled on ice for 2 hours and the HBsAg is removed by centrifugation.

5.) *Separation of two populations of HBsAg particles:* The HBsAg from the second PEG precipitation step is resuspended in distilled water in 1/20 of the original volume of plasma. The sample is adjusted to pH 5.0 and chilled on ice for 2 hours and stored overnight at 4°C. Centrifugation separates mostly small spherical particles in the supernatant and HBsAg particles enriched in filaments and Dane particles in the sediment. The pellet is suspended in distilled $H_2O$ to 1/40th the volume of the original plasma, solubilized by adjusting the pH 7, and immediately readjusted to pH 5. The sample is chilled on ice for 2 hours and recentrifuged. The supernatant (first wash) is aspirated and the precipitate is washed once more under the same conditions. Finally, the twice washed precipitate is resuspended in distilled $H_2O$ to 1/80th the volume of the original plasma and solubilized by adjusting the pH 7.0. This is product (A) in the purification summaries.

### B. Purification of HBsAg on hydroxylapatite

The HBsAg separated into pH 5 supernatant and first pH 5 wash supernatant in the previous step by PEG precipitation is further purified by batchwise adsorbtion to hydroxylapatite and subsequent elution with low ionic strength phosphate buffers as follows:

The pH 5 supernatant is pooled with the first wash of the pH 5 precipitate. The suspension is adjusted to pH 6.8 with 1 N HCl. 0.5 M sodium phosphate, pH 6.8 is added to a final concentration of 0.02 M and the volume is adjusted to 1/10 the volume of starting plasma.

An equal volume of packed hydroxylapatite (Bio-Rad Lab) prepared by low speed centrifugation (Sorvall RC—3 centrifuge, 2.000 rpm, 5 minutes) of a suspension is washed twice with two volumes of 0.02 M phosphate buffer, ph 6.8. The sediment of hydroxylapatite is resuspended in the total volume of sample and stirred on a magnetic stirrer for 2 hours at room temperature. The hydroxylapatite is sedimented by centrifugation (RC—3 centrifuge, 2,000 rpm, 5 minutes). The supernatant is decanted and the sediment is washed twice with 1/8 volume of starting plasma of 0.02 M and twice with 1/8 volume of starting plasma of 0.05 M Na phosphate buffer pH 6.8 with 2 hours of stirring at room temperature before each centrifugation.

The first supernatant and the 0.02 and 0.05 M Na phosphate eluates are clarified by centrifugation (RC—3 centrifuge, 3,000 rpm, 20 minutes). The clear supernatants are pooled and concentrated by ultrafiltration (Amicon, PM—30 membrane). The concentrated sample is washed twice in the Amicon concentrator with 0.02 M Na phosphate containing 0.02 percent $NaN_3$ adjusted to a final volume of 1/200th of the starting plasma of the same buffer. This is product (B) in the purification summaries.

### C. Purification of HBsAg by flotation isopycnic banding

The pool of small spherical particles purified by hydroxylapatite treatment and the preparation of HBsAg enriched in filaments and Dane particles from the pH 5.0 precipitation are pooled. Solid CsCl is added to a final density of 1.30 g/cc. The sample is distributed into centrifuge tubes to 40 percent of tube or rotor volume and overlayered with a layer of CsCl of 1.25 g/cc density corresponding to 20 percent of tube or rotor volume. The tubes or zonal rotor are then overlayered with a linear 1.25—1.05 g/cc CsCl gradient corresponding to 40 percent of tube or rotor volume. The samples are centrifuged for 24 hours at a g force corresponding to that obtained in an SW—40 rotor at 36,000 rpm.

The individual fractions are analyzed for proteins by $O.D._{280}$ at 1:10 dilution, for HBsAg by RIA at 1:1,000 and 1:10,000 dilution and for CsCl density by pyknometry. The fractions with a density of 1.17—1.25 g/cc containing HBsAg are pooled and adjusted to 1/100th the volume of the original plasma.

Results of Purification: Lots V and VI

*1. Yield*

The results of purification of lots V and VI are shown on Figs. 6—9. Recovery of HBsAg was 73.8 percent for Lot V and 78.0 percent for Lot VI. Yield of HBsAg (based on Kjeldahl) as 0.8 30 ug "doses"/ml starting plasma for Lot V and 1.54 $\mu$g "doses" for Lot VI.

2. Characterization of Product

*a) Purity*

To determine quantitatively the extent of contamination with serum proteins, the quantitative sensitivity of the agar gel diffusion test was first assessed with monospecific antisera (against the proteins known from previous studies to be possible contaminants) tested against dilutions of serum or plasma containing known contents of the various proteins (supplied by Behringwerke). The results are shown on Table 1.

Serial dilutions of the final isopycnic banding product, at protein contents (Kjeldahl) of 3.88 and 3.69 mg/ml, for Lots V and VI respectively, were then tested against each of the nine antisera. Albumin and IgG were detected in both preparations and, in addition, smaller quantities of prealbumin were detected in Lot V and C3 and $\alpha_2$-macroglobulin in Lot VI. The quantitative determination, summarized in Table 2, indicates that the contaminants constitute less than 5.5 percent of the total protein for Lot V and less than 5.2 percent for Lot VI.

*b) e Antigen*

e Antigen ($e_1$) was detected in the final product of Lot V by agar gel diffusion tests carried out in the laboratory. e Antigen was also detected in this preparation and in Lot VI by readioimmunoassay. However, e antigen was not detected in the final product of Lot VI by agar gel diffusion.

TABLE 1

*Sensitivity of Detection of Normal*
*Plasma Components by AGD*

| Proteins | Protein Content of Standards (mg/ml) | Sensitivity of Detection | |
|---|---|---|---|
| | | Dilution Endpoint | $\mu$g/ml |
| Prealbumin | 0.41 | 1:32—1:64 | 6.4—12.8 |
| Transferrin | 3.48 | 1:256—1:512 | 6.8—13.6 |
| IgG | 1.00 | 1:64—1:128 | 7.8—15.6 |
| Albumin | 51.00 | 1:6,400 | 8.0 |
| C3($\beta_1$ A Globulin) | 0.34 | 1:16—1:32 | 10.6—21.3 |
| Fibrinogen | 2.00 | 1:64—1:128 | 15.6—31.3 |
| $\alpha_2$-Macroglobulin | 0.70 | 1:16—1:32 | 21.9—43.8 |
| $\beta$-Lipoprotein | 4.26 | 1:64—1:128 | 33.3-66.6 |
| IgM | 1.16 | 1:16—1:32 | 36.3—72.5 |

### Table 2

*Impurities Detected in the Final Purified Preparation of HBsAg*

*A) Preparation: HBsAg — Lot V (3.88 mg/ml)*

| Impurities Detected | Sensitivity of Method ug/ml | Dilution Endpoint | Sample ug/ml | Percent of Total Protein |
|---|---|---|---|---|
| Albumin | 8.0 | 1:16 | 128.0 | 3.3 |
| Immune Globulin (IgG) | 7.8—15.6 | 1:4 | 31.2—64.4 | 0.8—1.6 |
| Prealbumin | 6.4—12.8 | 1:2 | 12.8—25.6 | 0.3—0.6 |
| Total | — | — | 171.0—215.0 | 4.4—5.5 |

*B) Preparation: HBsAg — Lot VI (3.69 mg/ml)*

| Albumin | 8.0 | 1:8 | 64.0 | 1.7 |
|---|---|---|---|---|
| Immune Globulin (IgG) | 7.8—15.6 | 1:4 | 31.2—62.4 | 0.8—1.6 |
| Complement (C3/C3c) | 10.6—21.3 | 1:1 | 10.6—21.3 | 0.3—0.6 |
| $\alpha_2$-Macroglobulin | 21.9—43.8 | 1:1 | 21.9—43.8 | 0.6—1.2 |
| Total | — | — | 127.7—191.4 | 3.5—5.2 |

*c) HB Core Antigen*

The final product of Lot V and Lot VI contained HBcAg as shown by radioimmunoassay.

*d) DNA Polymerase*

DNA polymerase was detected, though in small amount, in the main isopycnic banding fractions from Lot V and Lot VI: 265 and 125 CPM incorporated in the two samples respectively ($\geq$50 is positive).

Higher activities were found in the high density (1.26—1.4 g/cc) pools from the isopycnic banding step: 488 and 780 CPM, for Lots V and VI respectively.

*e) Morphology*

The particle distribution in the isopycnically banded HBsAg is illustrated in Figures 3 and 4, for Lots V and VI respectively. Dane particles and filaments are clearly present. It should be noted that these will subsequently be solubilized by Tween 80 during the inactivation process to reduce potential infectivity. The antigenic content of these particles should, however, be retained in the final product.

*4. Inactivation of Vaccine*

A series of experiments was carried out to define optimal conditions for exposure to detergent and formaldehyde during the final inactivation procedure. These experiments utilized a quantitative radioimmunoassay based on the linear relation (within the appropriate concentration range) between log dilution and Log CPM—B in the Ausria II radioimmunoassay (Figure 5). Test samples are compared to a highly purified frozen standard of homologous subtype. The method of calculation is illustrated on Table 3.

The following summarizes the results of our experiments on the inactivation procedure:

### Example 1

A small vaccine batch was prepared from Lot VI using one percent Tween 80 treatment in the presence of 0.5 mg/ml human serum albumin followed by Formalin 1:2000 for 3 days at 37°C. Following Formalin treatment and dialysis, the preparation was diluted to an estimated 50 ug/ml (based on quantitative RIA), sterile filtered, lyophilized and one vial rehydrated for assay. The results were as follows:

8

| | HBsAg by Quant. RIA |
|---|---|
| Starting Material | 466 $\mu$g/ml |
| FI + 1% Tween 80 I hr. room temperature | 418 $\mu$g/ml |
| FII + Sterile Filtration | 486 $\mu$g/ml |
| FIII + 1:2000 Formalin 1 day 37°C | 233 $\mu$g/ml |
| FIV + 1:2000 Formalin 2 days 37°C | 237 $\mu$g/ml |
| FV + 1:2000 Formalin 3 days 37°C | 276 $\mu$g/ml |
| FVI + 1:2000 Formalin 3 days 37°C + dialysis | 228 $\mu$g/ml |
| FVII FVI diluted to 50 $\mu$gm/ml | 59.5 $\mu$g/ml |
| FVIII FVII sterile filtered | 51.7 $\mu$g/ml |
| FIX FVIII Lyophilized and redissolved | 48.7 $\mu$g/ml |

Eighteen 5 ml vials are on hand from this experiment.

Table 3

*Calculation of Quantitative RIA for HBsAg*

| Sample | Dilution[1] | XCPM—B[2] | Log XCPM—B | $\log_{10}$ng/ml |
|---|---|---|---|---|
| HBsAg Standard | 1:800 | 12752 | 4.11 | 1.8 |
| | 1:1600 | 7273 | 3.86 | 1.5 |
| (50 $\mu$g/ml) | 1:3200 | 3385 | 3.53 | 1.2 |
| | 1:6400 | 1880 | 3.27 | 0.9 |
| | 1:12,800 | 964 | 2.98 | 0.6 |

Results of Linear regression analysis:
Correlation coefficient = 1.0
Slope = 0.95
$\log_{10}$ ng/ml corresponding to 10,000 CPM—B = 1.67
ng/ml corresponding to 10,000 CPM—B = 47.17

| | | | $\log_{10}$ dilution | |
|---|---|---|---|---|
| Lot VI | 1:32,000 | 19161 | 4.28 | 4.5 |
| Isopycnic Main Fraction | 1:64,000 | 11559 | 4.06 | 4.8 |
| | 1:128,000 | 6697 | 3.83 | 5.2 |
| | 1:256,000 | 2972 | 3.47 | 5.5 |
| | 1:512,000 | 1729 | 3.24 | 5.8 |

Correlation coefficient = 0.99
Slope = —0.81
$\log_{10}$ dilution corresponding to 10,000 CPM—B = 1:76,203
This dilution contains 47.17 ng/ml original sample contains 76,203 × 47.17
ng/ml = 3.59 mg/ml[3]

[1] Dilutions in normal human serum
[2] Mean of two tests less $\overline{X}$ of 7 negative controls (B)
[3] Kjeldahl indicated a protein content of 3.69 mg/ml

9

## Example 2

As the losses in the previous experiment (51 percent) appeared not to be reducible, it was decided to make a larger batch by the same procedure. As the method appears suitable for further use, it is described in detail.

60 mg Lot VI isopycinically banded HBsAg was diluted to 60 ml in 0.15 M NaCl, 0.1 M Phosphate buffer pH 7.2 (PBS) and 1 mg/ml final concentration human serum albumin (HSA). A sample was removed, diluted 1:20 in PBS, 1 mg/ml HSA (PBS—Alb) and frozen at —70°C for subsequent quantitative RIA (starting material). An equal volume of 2 percent Tween 80 in PBS was added and the solution held for one hour at room temperature. A sample was removed, diluted 1:10 in PBS-alb, and frozen as FI. The solution was then filtered through 0.45 and 0.22 micron millipore filters the FII samples was removed and frozen, and Formalin was added to a final concentration of 1:2000. The solution was placed at 37°C for 3 days, an aliquot (FV) removed for assay, and was then placed for dialysis against two changes (10 liters each) of 0.01 M Phosphate buffer pH 7.0 containing 1 part in 10,000 Thimerosol. An aliquot from the dialysed solution was removed as before (FVI) for quantitative RIA. On the basis of the results obtained, the solution was then adjusted to an estimated 50 µg/ml by dilution with 0.01 M phosphate buffer containing 0.5 mg/ml human serum albumin and 1:10,000 Thimerosol. A sample was removed as before (FVII) and the solution was sterile filtered (0.45 + 0.22 micron Millipore filters). Again a sample was removed (FVIII) and the solution was dispensed under sterile conditions in 5 ml volumes into 10 ml vaccine vials. These were frozen at —70°C and then lyophilized and capped. One vial was immediately rehydrated with 5 ml distilled water for assay (FIX).

*Yield:* 168 vials (stored at 4°C).

The results of this procedure were as follows:

| | HBsAg by Quant. RIA |
|---|---|
| Starting Material | 542.0 µg/ml |
| FI Starting Material + Tween 80 1 hour | 490.3 µg/ml |
| FII Starting Material + Tween 80 1 hour + sterile filtration | 490.3 µg/ml |
| FV FII + 1:2000 Formalin 3 days 37°C | 367.8 µg/ml |
| FVI FV + Dialysis | 325.0 µg/ml |
| FVII FVI diluted to "50 µg/ml" | 39.1 µg/ml |
| FVIII FVII + sterile filtration | 42.8 µg/ml |
| FIX FVIII lyophilized and redissolved | 35.1 µg/ml |

### 4. Immunogenicity in Guinea Pigs

As the inactivation procedure itself has been under investigation until very recently, extensive studies of immunogenicity in guinea pigs have been delayed. Present experience (Table 4) suggests that extinction of immunogenicity is observed with inocula containing about 0.2—2.0 µg HBsAg per guinea pig. There is clearly considerable variability in response between animals which will make it costly to obtain accurate estimates of immunogenicity. The results of an evaluation of vaccine prepared by Example 1 and starting material and FI samples from this preparation are presented on Table 5.

Again, the variation of response between different guinea pigs is evident. This is reminiscent of results which were obtained in chimpanzees with the Purcell vaccine in which about 1/4 of the chimpanzees appear to be hyporesponsive to immunization. The uniform response of animals inoculated with the HBV standard (laboratory standard of potency is 50 µg/ml) should not be interpreted as indicating that this material is more immunogenic than the Example 1 materials, since in the previous experiment (Table 4), the same standard gave non-uniform responses. Clearly, however, the material of Example 1 is immunogenic down to doses of 0.5—0.6 µg/guinea pig.

**0 005 864**

Table 4

*Summary of Guinea Pig Immunogenicity Tests*

| Example | Material | Dilution Inoculated | HBsAg $\mu$g/guinea pig | Proportion [1]seroconverting (Ausab) | $\overline{X}$ Ausab[2] Ratio Units |
|---|---|---|---|---|---|
| 3 | HBsAg purified by PEG + OH-apatite + 1 rate zonal separation | undil. | 20 | 2/2 | 72.1 |
| | | 1:4 | 5 | 2/2 | 14.9 |
| | | 1:16 | 1.2 | 1/2 | 23.7 |
| | | 1:64 | 0.3 | 1/2 | 2.2 |
| 4 | HBsAg standard purified as above plus 1 isopycnic centrifugation | undil. | 5 | 2/2 | 8.5 |
| | | 1:2 | 2.5 | 1/2 | 36.2 |
| | | 1:4 | 1.2 | 1/2 | 25.1 |
| | | 1:8 | 0.6 | 0/2 | — |
| 5 | Example 4 HBsAg purified by PEG, OH-apatite + isopycnic flotation | undil. | 1.57 | 2/3 | 5.4 |
| | | 1:2 | 0.79 | 3/4 | 3.7 |
| | | 1:4 | 0.39 | 0/4 | — |
| 6 | above + 0.1% DOCA | undil. | 0.9 | 1/4 | 2.1 |
| | | 1:2 | 0.45 | 1/4 | 7.1 |
| | | 1:4 | 0.23 | 2/4 | 5.1 |
| | | 1:8 | 0.12 | 1/4 | 2.3 |

[1] Proportion with positive Ausab (>2.1 Ratio units) after 3 weeks after 1.M. inoculation
[2] Mean of responders

Table 5

Guinea Pig Immunogenicity of Vaccine of Example 1

| Material | Dilution Inoculated | Volume (ml) Inoculated | HBsAg $\mu$g/guinea pig | Proportion seroconverting (Ausab)[1] | $\overline{X}$ Ausab Ratio Units[2] |
|---|---|---|---|---|---|
| HBV standard | undil. | 0.2 | 10 | 4/4 | 18/1 |
| | undil. | 0.1 | 5 | 4/4 | 20.0 |
| | 1:2 | 0.1 | 2.5 | 4/4 | 12.7 |
| | 1:4 | 0.1 | 1.2 | 4/4 | 3.1 |
| Example 1 | undil. | 0.1 | 4.6 | 4/4 | 27.3 |
| Starting Material | 1:2 | 0.1 | 2.7 | 1/4 | 43.5 |
| | 1:4 | 0.1 | 1.1 | 0/3 | — |
| | 1:8 | 0.1 | 0.5 | 3/4 | 7.8 |
| Example 1 Fl | undil. | 0.1 | 4.1 | 3/4 | 24.5 |
| (Tween 80 treated) | 1:2 | 0.1 | 2.0 | 2/4 | 25.9 |
| | 1:4 | 0.1 | 1.0 | 1/4 | 14.8 |
| | 1:8 | 0.1 | 0.5 | 2/3 | 6.9 |
| Example 1 | undil. | 0.2 | 9.6 | 3/4 | 13.3 |
| Final Vaccine Lyophilized and Rehydrated | undil. | 0.1 | 4.8 | 1/4 | 27.4 |
| | 1:2 | 0.1 | 2.4 | 2/4 | 10.6 |
| | 1:4 | 0.1 | 1.2 | 3/4 | 6.8 |
| | 1:8 | 0.1 | 0.6 | 1/4 | 8.0 |

[1] Proportion with positive Ausab (>2.1 Ratio Units) 4 weeks pinoc.
[2] Mean of responders (>2.1 ratio units)

**Claims for Contracting States: BE DE FR GB NL SE**

1. An antigenic composition comprising a subparticulate mass having a particle size less than 20 nm, said mass containing hepatitis B surface antigen (HBsAg).

2. An antigenic composition according to claim 1 wherein said mass contains e-antigen.

3. An antigenic composition according to claims 1 or 2 wherein said subparticulate mass has a particle size less than 16 nm, preferably less than 5 nm.

4. An antigenic composition according to claim 3 wherein said subparticulate mass has a particle size of between 1 and 4 nm.

5. An antigenic composition according to anyone of claims 1—4 wherein said antigenic composition is substantially free of detectable DNA, and/or DNA polymerase, and/or hepatitis B core antigen.

6. An antigenic composition according to anyone of claims 1—5 wherein said antigenic composition contains fragments of Dane particles and filaments.

7. An antigenic composition according to anyone of claims 1—6 wherein said composition is substantially free of antibody to hepatitis B surface antigen (anti-HBs) and/or of antibody to e-antigen (anti-HBe).

8. An antigenic composition according to anyone of claims 1—7 wherein said subparticulate

12

mass has an aqueous density of between 1.21 and 1.24 g/cc determined by isopycnic banding in CsCl gradients.

9. An antigenic composition according to anyone of claims 1—8 wherein said subparticulate mass has a molecular weight in the range of 20,000 to 1,000,000 Daltons.

10. An antigenic composition according to anyone of claims 1—9 wherein said subparticulate mass is present in said antigenic composition in an amount of at least 0.001 weight percent, preferably in an amount of between 0.001 and 0.01 weight percent.

11. An antigenic composition according to claim 10 wherein said subparticulate mass is present in said antigenic composition in an amount sufficient to effect an antibody response when administered to an animal.

12. An antigenic composition according to anyone of claims 1—11 wherein said antigenic mass additionally contains a physiologically acceptable medium.

13. An antigenic composition according to anyone of claims 1—12 wherein said subparticulate mass has a generally spherical morphology.

14. An antigenic composition according to anyone of claims 1 to 13, wherein said antigenic composition is obtained by contacting an antigenic mass containing particles of HBsAg of particle size of at least 20 nm, which particles also contain said e-antigen with a detergent in an amount of 0.1 to 2.0 weight percent, based upon the weight of said particles of size at least 20 nm for at least 0.1 hours and thereafter contacting the so-treated antigenic composition with an aqueous aldehyde solution.

15. An antigenic composition according to claim 14, wherein the antigenic composition of particles of at least 20 nm is contacted with said detergent for a period of time of at least one day, preferably for a period of time between one day and 10 days.

16. An antigenic composition according to claim 14 or 15 wherein the concentration of said detergent is between 0.5 and 1.0 weight percent.

17. An antigenic composition according to anyone of claims 14 to 16, wherein said detergent is a physiologically acceptable non-ionic detergent and the detergent contact is effected at a temperature of between 4 and 90°C.

18. An antigenic composition according to anyone of claims 14 to 17, wherein following the contact of said antigenic mass containing particles of at least 20 nm with said detergent, the resultant mass is subjected to sterile filtration and, following contact with said aldehyde, preferably at a temperature between 4 and 90°C for between one and 10 days, the resultant so-treated mass is subjected to diafiltration.

19. A process for preparing an antigenic composition according to anyone of claims 1 to 18 which comprises contacting an antigenic mass containing particles of HBsAg of particle size of at least 20 nm with a detergent.

20. A process according to claim 19 wherein said detergent is a physiologically acceptable nonionic or ionic detergent.

21. A process according to claims 19 or 20 wherein the contacting of said antigenic mass with said detergent is performed for 1—4 hours.

22. A process according to anyone of claims 19—21 wherein the contacting of said antigenic mass with said detergent is performed for at least one hour at a temperature of 4 to 90°C.

23. A process according to anyone of claims 19—22 wherein said contacting is performed using a detergent in an amount of 0.1 to 2.0 weight percent, based upon the weight of said particles of size at least 20 nm.

24. A process according to claim 23 wherein said detergent is present in an aqueous solution in a concentration of between 0.5 and 1.0 weight percent.

25. A process according to anyone of claims 19—24 wherein (A) said antigenic mass containing particles of a size of at least 20 nm is initially contacted with said detergent for a time of between 0,1 and 4.0 hours, especially between 1.0 and 4.0 hours and (B) thereafter the detergent containing antigenic composition is contacted with an aqueous aldehyde solution.

26. A process according to claim 25 wherein step B is performed by maintaining said detergent containing antigenic composition in contact with said aldehyde at a temperature of between 4 and 90°C, preferably between 4 and 37°C for between 1 and 10 days.

27. A process according to anyone of claims 19 to 26 wherein said detergent is selected from the group consisting of polyoxyethylene derivatives of fatty acid partial esters of sorbitol anhydrides, such as a polyoxyethylene derivative of sorbitan monooleate or monolaurate; sodium deoxycholate; synthetic zwitterionic sulfobetaine detergents such as N-dodecyl-N,N-dimethyl-2-ammonio-1-ethane sulfonate and its congeners; and octyl-β-D-glucopyranosid.

28. A process according to claims 25 or 26 wherein following step A, the resultant mass is subjected to sterile filtration and following step B, the resultant mass is subjected to diafiltration.

29. A process according to claim 28 wherein following said diafiltration the resultant mass is diluted to an HBsAg concentration of 10 to 100 ugm/ml and the mass is phosphate buffered to a pH of 7.0 ± 0.5.

30. A process according to claim 29 wherein following the buffering the resultant mass is subjected to sterile filtration, filling and lyophilization.

# 0 005 864

31. A process according to anyone of claims 19—30 wherein the antigenic mass subjected to detergent treatment comprises HBsAg containing particles whose particle size is 16 to 50 nm.

32. A process according to anyone of claims 19—31 wherein at least some of the particles contacted with said detergent contain e-antigen and/or are Dane particles or filaments.

## Revendications pour les Etats contractants: BE DE FR GB NL + SE

1. Composition antigénique comprenant une mase subparticulaire ayant une dimension de particule inférieure à 20 nm, ladite masse contenant de l'antigène de surface d'hépatite B (HBsAg).

2. Composition antigénique selon la revendication 1, dans laquelle ladite masse contient de l'antigène-e.

3. Composition antigénique selon la revendication 1 ou 2, dans laquelle ladite masse subparticulaire présente des dimensions de particules inférieures à 16 nm, de préférence inférieures à 5 nm.

4. Composition antigénique selon la revendication 3, dans laquelle la masse subparticulaire présente des dimensions de particules comprises entre 1 et 4 nm.

5. Composition antigénique selon l'une des revendications 1 à 4, pratiquement exempte d'ADN, et/ou d'ADN polymérase.

6. Composition antigénique selon l'une des revendications 1 à 5, qui contient des fragments de particules de Dane et des filaments.

7. Composition antigénique selon une des revendications 1 à 6, pratiquement exempte d'anticorps d'antigène de surface d'hépatite B (anti-HBs) et-ou d'anticorps d'antigène-e (anti-HBe).

8. Composition antigénique selon une des revendications 1 à 7, dans laquelle la masse subparticulaire a une densité par rapport à l'eau comprise entre 1,21 et 1,24 g/ml, déterminée par formation de bandes isopycniques dans un gradient de CsCl.

9. Composition antigénique selon l'une des revendications 1 à 8, dans laquelle la masse subparticulaire a un poids moléculaire compris entre 20 000 et 1 000 000 Daltons.

10. Composition antigénique selon une des revendications 1 à 9, dans laquelle la masse subparticulaire est présente en quantité d'au moins 0,001% en poids, de préférence en quantité comprise entre 0,001 et 0,01% en poids.

11. Composition antigénique selon la revendication 10, dans laquelle la masse subparticulaire est présente en quantité suffisante pour provoquer une réponse danticorps lors de son administration à un animal.

12. Composition antigénique selon l'une des revendications 1 à 11, dans laquelle ladite masse antigénique contient en outre un milieu physiologiquement acceptable.

13. Composition antigénique selon une des revendications 1 à 12, dans laquelle ladite masse subparticulaire présente une morphologie généralement sphérique.

14. Composition antigénique selon une des revendications 1 à 13, obtenue par mise en contact d'une masse antigénique contenant des particules de HBsAg de dimension au moins égale à 20 nm, lesdites particules renfermant également de l'antigène-e avec un détergent en quantité de 0,1 à 2% en poids, rapporté au poids des particules de dimension au moins égale à 20 nm, pendant au moins 6 minutes, puis mise en contact ultérieure de la composition antigénique ainsi traitée avec une solution aqueuse d'aldéhyde.

15. Composition antigénique selon la revendication 14, dans laquelle la composition antigénique de particules d'au moins 20 nm est mise en contact avec le détergent pendant une durée d'au moins 1 jour, de préférence pendant une durée comprise entre 1 et 10 jours.

16. Composition antigénique selon la revendication 14 ou 15, dans laquelle la concentration de détergent est comprise entre 0,5 et 1% en poids.

17. Composition antigénique selon une des revendications 14 à 16, dans laquelle le détergent est un détergent ionique ou non ionique, physiologiquement acceptable, sa mise en contact étant réalisée à une température comprise entre 4° et 90°C.

18. Composition antigénique selon une des revendications 14 à 17, pour laquelle après la mise en contact de la masse antigénique contenant les particules d'au moins 20 nm, avec le détergent, la masse résultante est soumise à une filtration stérile, et, après la mise en contact avec l'aldéhyde, de préférence à une température comprise entre 4° et 90°C, pendant 1 à 10 jours, la masse résultante ainsi traitée est soumise à une diafiltration.

19. Procédé de préparation d'une composition antigénique selon une des revendications 1 à 18, comprenant la mise en contact d'une masse antigénique contenant des particules de HBsAg de dimension au moins égale à 20 nm, avec un détergent.

20. Procédé selon la revendication 19, dans lequel le détergent est un détergent ionique ou non ionique, physiologiquement acceptable.

21. Procédé selon la revendication 19 ou 20, dans lequel la mise en contact de la masse antigénique avec le détergent est réalisée pendant 1 à 4 heures.

22. Procédé selon une des revendications 19 à 21, dans lequel la mise en contact de la masse antigénique avec le détergent, s'effectue pendant au moins 1 heure à une température de 4° à 90°C.

14

**0 005 864**

23. Procédé selon une des revendications 19 à 22, dans lequel la quantité de détergent mis en contact avec les particules d'au moins 20 nm de dimension, représente 0,1 à 2% en poids, rapporté au poids des particules.

24. Procédé selon la revendication 23, dans lequel le détergent est présent dans une solution aqueuse à une concentration comprise entre 0,5 et 1% en poids.

25. Procédé selon une des revendications 19 à 24, dans lequel A) la masse antigénique contenant des particules d'au moins 20 nm est mise initialement en contact avec le détergent pendant une durée comprise entre 6 minutes et 4 heures, en particulier entre 1 et 4 heures, et B) ensuite, la composition antigénique contenant le détergent est mise en contact avec une solution aqueuse d'aldéhyde.

26. Procédé selon la revendication 25, dans lequel le stade B est réalisé par maintien de la composition antigénique contenant le détergent, en contact avec l'aldéhyde, à une température comprise entre 4° et 37°C, pendant 1 à 10 jours.

27. Procédé selon une des revendications 19 à 26, dans lequel le détergent est choisi dans le groupe comprenant les dérivés polyoxyéthyléniques d'esters partiels d'acide gras et d'anhydrides de sorbitol, tels qu'un dérivé polyoxyéthylénique de monooléate ou monolaurate de sorbitane, le désoxycholate de sodium; les détergents de sulfobétaïne amphotères synthétiques comme le sulfonate de N-dodécyl N,N-diméthyl ammonio-2 éthane et ses homologues; et l'octyl $\beta$-D-glucopyranoside.

28. Procédé selon la revendication 25 ou 26, dans lequel après le stade A, la masse résultante est soumise à une filtration stérile, et après le stade B, la masse résultante est soumise à une diafiltration.

29. Procédé selon la revendication 28, dans lequel après la diafiltration, la masse résultante est diluée à une concentration en HBsAg de 10 à 100 $\mu$g/ml, et la masse obtenue est tamponnée avec du phosphate à un pH de 7 $\pm$ 0,5.

30. Procédé selon la revendication 29, dans lequel après avoir été tamponnée, la masse résultante est soumise aux opérations de filtration stérile, remplissage et lyophilisation.

31. Procédé selon une des revendications 19 à 30, dans lequel la masse antigénique soumise au traitement par le détergent comprend des particules contenant HBsAg, dont la dimension va de 16 à 50 nm.

32. Procédé selon une des revendications 19 à 31, dans lequel au moins certaines des particules mises en contact avec le détergent, contiennent de l'antigène-e, et/ou des particules de Dane ou des filaments.

**Patentansprüche für die Vertragsstaaten: BE DE FR GB NL SE**

1. Antigen-Zusammensetzung, enthaltend eine subpartikulare Masse mit einer Teilchengröße von weniger als 20 nm, wobei die Masse Hepatitis-B-Oberflächenantigen (HBsAg) enthält.

2. Antigen-Zusammensetzung nach Anspruch 1, dadurch gekennzeichnet, daß die Masse e-Antigen enthält.

3. Antigen-Zusammensetzung nach Anspruch 1 oder 2, dadurch gekennzeichnet, daß die subpartikulare Masse eine Teilchengröße von weniger als 16 nm, vorzugsweise von weniger als 5 nm, hat.

4. Antigen-Zusammensetzung nach Anspruch 3, dadurch gekennzeichnet, daß die subpartikulare Masse eine Teilchengröße zwischen 1 und 4 nm hat.

5. Antigen-Zusammensetzung nach einem der Ansprüche 1 bis 4, dadurch gekennzeichnet, daß die Antigen-Zusammensetzung praktisch frei von nachweisbarer DNS und/oder DNS-Polymerase und/oder Hepatitis-B-Kernantigen ist.

6. Antigen-Zusammensetzung nach einem der Ansprüche 1 bis 5, dadurch gekennzeichnet, daß die Antigen-Zusammensetzung Fragmente von Dane-Teilchen und -Filamenten enthält.

7. Antigen-Zusammensetzung nach einem der Ansprüche 1 bis 6, dadurch gekennzeichnet, daß die Zusammensetzung praktisch frei von Antikörpern gegen Hepatitis-B-Oberflächenantigen (anti-HBs) und/oder von Antikörpern gegen e-Antigen (anti-HBe) ist.

8. Antigen-Zusammensetzung nach einem der Ansprüche 1 bis 7, dadurch gekennzeichnet, daß die subpartikulare Masse eine Dichte (im wäßrigen Medium) zwischen 1,21 und 1,24 g/cm³ (bestimmt nach isopyknischen Banden in CsCl-Gradienten) hat.

9. Antigen-Zusammensetzung nach einem der Ansprüche 1 bis 8, dadurch gekennzeichnet, daß die subpartikulare Masse ein Molekulargewicht im Bereich von 20 000 bis 1 000 000 Dalton hat.

10. Antigen-Zusammensetzung nach einem der Ansprüche 1 bis 9, dadurch gekennzeichnet, daß die subpartikulare Masse in der Antigen-Zusammensetzung in einer Menge von mindestens 0,001 Gew.-%, vorzugsweise in einer Menge zwischen 0,001 und 0,01 Gew.-% vorliegt.

11. Antigen-Zusammensetzung nach Anspruch 10, dadurch gekennzeichnet, daß die subpartikulare Masse in der Antigen-Zusammensetzung in einer solchen Menge vorhanden ist, daß bei einer Verabereichung an ein Lebewesen eine Antikörperrreaktion erfolgt.

12. Antigen-Zusammensetzung nach einem der Ansprüche 1 bis 11, dadurch gekennzeichnet, daß die antigene Masse zusätzlich ein physiologisch verträgliches Medium enthält.

13. Antigen-Zusammensetzung nach einem der Ansprüche 1 bis 12, dadurch gekennzeichnet, daß die subpartikulare Masse eine im allgemeinen sphärische Morphologie hat.

15

14. Antigen-Zusammensetzung nach einem der Ansprüche 1 bis 13, dadurch gekennzeichnet, daß sie erhalten wird, indem eine antigene Masse mit HBsAg-Teilchen mit einer Teilchengröße von mindestens 20 nm, wobei diese Teilchen auch das e-Antigen enthalten, mit einem Detergens in einer Menge von 0,1 bis 2,0 Gew.-%, bezogen auf das Gewicht der Teilchen mit einer Größe von mindestens 20 nm, mindestens 0,1 Stunde in Berührung gebracht wird, worauf die so behandelte Antigen-Zusammensetzung mit einer wäßrigen Aldehydlösung in Berührung gebracht wird.

15. Antigen-Zusammensetzung nach Anspruch 14, dadurch gekennzeichnet, daß die Antigen-Zusammensetzung mit einer Teilchengröße von mindestens 20 nm mindestens einen Tag, vorzugsweise über einen Zeitraum von 1 bis 10 Tagen, mit dem Detergens in Berührung gebracht wird.

16. Antigen-Zusammensetzung nach Anspruch 14 oder 15, dadurch gekennzeichnet, daß die Konzentration des Detergens zwischen 0,5 und 1,0 Gew.-% liegt.

17. Antigen-Zusammensetzung nach einem der Ansprüche 14 bis 16, dadurch gekennzeichnet, daß das Detergens ein physiologisch verträgliches, nicht-ionisches oder ionisches Detergens ist und die Berührung mit dem Detergens bei einer Temperatur zwischen 4 und 90°C bewirkt wird.

18. Antigen-Zusammensetzung nach einem der Ansprüche 14 bis 17, dadurch gekennzeichnet, daß nach dem Kontakt der antigenen Masse mit Teilchen von mindestens 20 nm mit dem Detergens die erhaltene Masse steril filtriert wird und nach der Berührung mit dem Aldehyd, vorzugsweise bei einer Temperatur zwischen 4 und 90° über 1 bis 10 Tage, die so behandelte, erhaltene Masse einer Diafiltration unterzogen wird.

19. Verfahren zur Herstellung einer Antigen-Zusammensetzung nach einem der Ansprüche 1 bis 18, dadurch gekennzeichnet, daß man eine antigene Masse, die HBsAg-Teilchen mit einer Teilchengröße von mindestens 20 nm enthält, mit einem Detergens in Berührung bringt.

20. Verfahren nach Anspruch 19, dadurch gekennzeichnet, daß das Detergens ein physiologisch verträgliches, nicht-ionisches oder ionisches Detergens darstellt.

21. Verfahren nach Anspruch 19 bis 21, dadurch gekennzeichnet, daß der Kontakt zwischen der antigenen Masse und dem Detergens über einen Zeitraum von 1 bis 4 Stunden erfolgt.

22. Verfahren nach einem der Ansprüche 19 bis 21, dadurch gekennzeichnet, daß der Kontakt der antigenen Masse mit dem Detergens mindestens 1 Stunde bei einer Temperatur von 4 bis 90°C erfolgt.

23. Verfahren nach einem der Ansprüche 19 bis 22, dadurch gekennzeichnet, daß der Kontakt unter Verwendung eines Detergens in einer Menge von 0,1 bis 2,0 Gew.-%, bezogen auf das Gewicht der Teilchen mit einer Größe von mindestens 20 nm, erfolgt.

24. Verfahren nach Anspruch 23, dadurch gekennzeichnet, daß das Detergens in einer wäßrigen Lösung in einer Konzentration zwischen 0,5 und 1,0 Gew.-% vorhanden ist.

25. Verfahren nach einem der Ansprüche 19 bis 24, dadurch gekennzeichnet, daß (A) die antigene Masse, die Teilchen mit einer Größe von mindestens 20 nm enthält, zunächst über einen Zeitraum zwischen 0,1 und 4,0 Stunden, insbesondere zwischen 1,0 und 4,0 Stunden mit dem Detergens in Berührung gebracht wird und (B) dann die das Detergens enthaltende Antigen-Zusammensetzung mit einer wäßrigen Aldehydlösung in Berührung gebracht wird.

26. Verfahren nach Anspruch 25, dadurch gekennzeichnet, daß die Stufe (B) durchgeführt wird, indem die das Detergens enthaltende Antigen-Zusammensetzung mit dem Aldehyd bei einer Temperatur zwischen 4 und 90°C, vorzugsweise zwischen 4 und 37°C, über 1 bis 10 Tage in Berührung gehalten wird.

27. Verfahren nach einem der Ansprüche 19 bis 26, dadurch gekennzeichnet, daß das Detergens aus der Gruppe der Polyoxyäthylenderivate von Fettsäure-Partialestern von Sorbit-Anhydriden, z.B. einem Polyoxyäthylenderivat von Sorbitan-monooleat oder -monolaurat; Natriumdeoxycholat; synthetischen zwitterionischen Sulfobetain-Detergentien, wie N-Dodecyl-N,N-dimethyl-2-ammonio-1-äthansulfonat und verwandten Verbindungen; und Octyl-$\beta$-D-glucopyranosid ausgewählt ist.

28. Verfahren nach Anspruch 25 oder 26, dadurch gekennzeichnet, daß die nach der Stufe A erhaltene Masse steril filtriert und die nach der Stufe B erhaltene Masse einer Diafiltration unterzogen wird.

29. Verfahren nach Anspruch 28, dadurch gekennzeichnet, daß die nach der Diafiltration erhaltene Masse bis auf eine HBsAg-Konzentration von 10 bis 100 $\mu$g/ml verdünnt und mit Phosphat bis auf einen pH-Wert von 7,0 $\pm$ 0,5 gepuffert wird.

30. Verfahren nach Anspruch 29, dadurch gekennzeichnet, daß die nach der Pufferung erhaltene Masse steril filtriert, abgefüllt und lyophilisiert wird.

31. Verfahren nach einem der Ansprüche 19 bis 30, dadurch gekennzeichnet, daß die der Detergens-Behandlung unterzogene Masse HBsAg-haltige Teilchen mit einer Teilchengröße von 16 bis 50 nm enthält.

32. Verfahren nach einem der Ansprüche 19 bis 31, dadurch gekennzeichnet, daß mindestens ein Teil der mit dem Detergens behandelten Teilchen e-Antigen enthält und/oder Dane-Teilchen oder -Filamente darstellt.

**Claims for Contracting State: AT**

1. A process for preparing an antigenic composition comprising a subparticulate mass having a

16

particle size less than 20 nm, said mass containing hepatitis B surface antigen (HBsAg), which comprises contacting an antigenic mass containing particles of HBsAg of a particle size of at least 20 nm with a detergent.

2. A process according to claim 1, wherein said detergent is a physiologically acceptable non-ionic or ionic detergent.

3. A process according to claims 1 or 2, wherein the contacting of said antigenic mass with said detergent is performed for 1—4 hours.

4. A process according to anyone of claims 1 to 3, wherein the contacting of said antigenic mass with said detergent is performed for at least one hour at a temperature of 4 to 90°C.

5. A process according to anyone of claims 1 to 4, wherein said contacting is performed using a detergent in an amount of 0.1 to 2.0 weight percent, based upon the weight of said particles of a size at least 20 nm.

6. A process according to claim 5, wherein said detergent is present in an aqueous solution in a concentration of between 0.5 and 1.0 weight percent.

7. A process according to anyone of claims 1 to 6, wherein A said antigenic mass containing particles of a size of at least 20 nm is initially contacted with said detergent for a time of between 0.1 and 4.0 hours, especially between 1.0 and 4.0 hours and B thereafter the detergent containing antigenic composition is contacted with an aqueous aldehyde solution.

8. A process according to claim 19, wherein step B is performed by maintaining said detergent containing antigenic composition in contact with said aldehyde at a temperature of between 4 and 90°C, preferably between 4 and 37°C for between 1 and 10 days.

9. A process according to anyone of claims 1 to 8 wherein said detergent is selected from the group consisting of polyoxyethylene derivatives of fatty acid partial esters of sorbitol anhydrides, such as a polyoxyethylene derivative of sorbitan monooleate or monolaurate; sodium deoxycholate; synthetic zwitterionic sulfobetaine detergents such as N-dodecyl-N,N-dimethyl-2-ammonio-1-ethane sulfonate and its congeners; and octyl-$\beta$-D-glucopyranosid.

10. A process according to claims 7 or 8, wherein following step A, the resultant mass is subjected to sterile filtration and following step B, the resultant mass is subjected to diafiltration.

11. A process according to claim 10 wherein following said diafiltration the resultant mass is diluted to an HBsAg concentration of 10 to 100 ugm/ml and the mass is phosphate buffered to a pH of $7.0 \pm 0.5$.

12. A process according to claim 11 wherein following the buffering the resultant mass is subjected to sterile filtration, filling and lyophilization.

13. A process according to anyone of claims 1 to 12 wherein the antigenic mass subjected to detergent treatment comprises HBsAg containing particles whose particle size is 16 to 50 nm.

14. A process according to anyone of claims 1 to 13, wherein at least some of the particles contacted with said detergent contain e-antigen and/or are Dane particles or filaments.

15. A process according to anyone of claims 1 to 14, wherein the subparticulate mass obtained after treatment with said detergent has a particle size less than 16 nm, preferably less than 5 $\mu$m, most preferably between 1 and 4 nm.

16. A process according to anyone of claims 1 to 15 wherein said antigenic composition is substantially free of detectable DNA, and/or DNA polymerase, and/or hepatitis B core antigen.

17. A process according to anyone of claims 1 to 16, wherein said antigenic composition is substantially free of antibody to hepatitis B surface antigen (anti-HBs) and/or of antibody to e-antigen (anti-HBe).

18. A process according to anyone of claims 1 to 17, wherein the subparticulate mass obtained after treatment with said detergent has an aqueous density of between 1.21 and 1.24 g/cc determined by isopycnic banding in CsCl gradients.

19. A process according to anyone of claims 1 to 18, wherein the subparticulate mass obtained after treatment with said detergent has a molecular weight in the range of 20 000 to 1 000 000 Daltons.

20. A process according to anyone of claims 1 to 19, wherein the subparticulate mass obtained after treatment with said detergent is present in said antigenic composition in an amount of at least 0.001 weight percent, preferably in an amount of between 0.001 and 0.01 weight percent.

21. A process according to anyone of claims 1 to 20, wherein the subparticulate mass obtained after treatment with said detergent is present in said antigenic composition in an amount sufficient to effect an antibody response when administered to an animal.

22. A process according to anyone of claims of claims 1 to 21, wherein said antigenic mass additionally is mixed with a physiologically acceptable medium.

23. A process according to anyone of claims 1 to 22, wherein the subparticulate mass obtained after treatment with said detergent has a generally spherical morphology.

**Revendications pour l'Etat contractant: AT**

1. Procédé de préparation d'une composition antigénique comprenant une masse subparticulaire de dimension de particule inférieure à 20 nm, ladite masse contenant de l'antigène de surface d'hépatite B (HBsAg), qui consiste à mettre en contact une masse antigénique contenant des particules de HBsAg dont la dimension est d'au moins 20 nm, avec un détergent.

2. Procédé selon la revendication 1, dans lequel le détergent est un détergent ionique ou non ionique, physiologiquement acceptable.

3. Procédé selon la revendication 1 ou 2, dans lequel la mise en contact de la masse antigénique avec le détergent a lieu pendant 1 à 4 heures.

4. Procédé selon une des revendications 1 à 3, dans lequel la mise en contact de la masse antigénique avec le détergent, s'effectue pendant au moins 1 heure, à une température de 4° à 90°C.

5. Procédé selon une des revendications 1 à 4, dans lequel la quantité de détergent mis en contact représente 0,1 à 2% en poids, rapporté au poids des particules d'au moins 20 nm.

6. Procédé selon la revendication 5, dans lequel le détergent est présent sous forme de solution aqueuse à la concentration de 0,5 à 1% en poids.

7. Procédé selon l'une des revendications 1 à 6, dans lequel A) la masse antigénique contenant les particules d'au moins 20 nm est initialement mise en contact avec le détergent pendant une durée comprise entre 6 minutes et 4 heures, en particulieur 1 et 4 heures, et B) la composition antigénique contenant le détergent est ensuite mise en contact avec une solution aqueuse d'aldéhyde.

8. Procédé selon la revendication 7, dans lequel le stade B est réalisé par maintien en contact de la composition antigénique contenant le détergent, avec l'aldéhyde, à une température comprise entre 4° et 90°C, de préférence entre 4° et 37°C, pendant 1 à 10 jours.

9. Procédé selon l'une des revendications 1 à 8, dans lequel le détergent est choisi parmi le groupe comprenant les dérivés polyoxyéthyléniques d'esters partiels d'acide gras et d'anhydrides de sorbitol, tels qu'un dérivé polyoxyéthylénique de monooléate ou monolaurate ce sorbitane, le désoxycholate de sodium, les détergents de sulfobétaïne amphotères synthétiques comme le sulfonate de N-dodécyl N,N-diméthyl ammono-2 éthane et ses homologues et l'octyl $\beta$-D-glucopyranoside.

10. Procédé selon la revendication 7 ou 8, dans lequel après le stade A, la masse résultante est soumise à une filtration stérile, et après le stade B, la masse résultante est soumise à une diafiltration.

11. Procédé selon la revendication 10, dans lequel après la diafiltration, la masse résultante est diluée à une concentration en HBsAg de 10 à 100 $\mu$g/ml, et cette masse est tamponnée avec du phosphate à pH 7 $\pm$ 0,5.

12. Procédé selon la revendication 11, dans lequel après avoir été tamponnée, la masse résultante est soumise aux opérations de filtration stérile, remplissage et lyophilisation.

13. Procédé selon une des revendications 1 à 12, dans lequel la masse antigénique soumise au traitement par le détergent, comprend des particules contenant HBsAg, dont la dimension va de 16 à 50 nm.

14. Procédé selon l'une des revendications 1 à 13, dans lequel au moins quelques unes des particules mises en contact avec le détergent, contiennent de l'antigène-e, et/ou des particules de Dane ou des filaments.

15. Procédé selon une des revendications 1 à 14, dans lequel la masse subparticulaire obtenue après traitement avec le détergent, a une dimension de particule inférieure à 16 nm, de préférence inférieure à 5 nm, au mieux comprise entre 1 et 4 nm.

16. Procédé selon une des revendications 1 à 15, dans lequel la composition antigénique est pratiquement exempte d'ADN, et/ou d'ADNpolymérase, et/ou d'antigène de noyau d'hépatite B.

17. Procédé selon une des revendications 1 à 16, dans lequel la composition antigénique est pratiquement exempte d'anticorps d'antigène de surface d'hépatite B (anti-HBs) et/ou d'anti-corps d'antigène-e (anti-HBe).

18. Procédé selon une des revendications 1 à 17, dans lequel la masse subparticulaire obtenue après traitement avec le détergent, présente une densité aqueuse comprise entre 1,21 et 1,24 g/ml, déterminée par formation de bandes isopycniques dans un gradient de CsCl.

19. Procédé selon une des revendications 1 à 18, dans lequel la masse subparticulaire obtenue après traitement avec le détergent, a un poids moléculaire compris entre 20 000 et 1 000 000 Daltons.

20. Procédé selon une des revendications 1 à 19, dans lequel la masse subparticulaire obtenue après traitement avec le détergent, est présente dans la composition antigénique à raison d'au moins 0,001% en poids, de préférence entre 0,001 et 0,01% en poids.

21. Procédé selon une des revendications 1 à 20, dans lequel la masse subparticulaire obtenue après traitement avec le détergent, est présente dans la composition antigénique en quantité suffisante pour entrainer une réponse d'anticorps, lors de l'administration à un animal.

22. Procédé selon une des revendications 1 à 21, dans lequel la masse antigénique est en outre mélangée avec un milieu physiologiquement acceptable.

23. Procédé selon une des revendications 1 à 22, dans lequel la masse subparticulaire obtenue après traitement avec le détergent, présente une morphologie généralement sphérique.

**Patentansprüche für den Vertragsstaat: AT**

1. Verfahren zur Herstellung einer Antigen-Zusammensetzung, enthaltend eine subpartikulare

# 0 005 864

Masse mit einer Teilchengröße von weniger als 20 nm, wobei die Masse Hepatitis-B-Oberflächenantigen (HBsAg) enthält, dadurch gekennzeichnet, daß man eine antigene Masse, die HBsAg-Teilchen mit einer Teilchengröße von mindestens 20 nm enthält, mit einem Detergens in Berührung bringt.

2. Verfahren nach Anspruch 1, dadurch gekennzeichnet, daß das Detergens ein physiologisch verträgliches, nicht-ionisches oder ionisches Detergens darstellt.

3. Verfahren nach Anspruch 1 oder 2, dadurch gekennzeichnet, daß der Kontakt zwischen der antigenen Masse und dem Detergens über einen Zeitraum von 1 bis 4 Stunden erfolgt.

4. Verfahren nach einem der Ansprüche 1 bis 3, dadurch gekennzeichnet, daß der Kontakt der antigenen Masse mit dem Detergens mindestens 1 Stunde bei einer Temperatur von 4 bis 90°C erfolgt.

5. Verfahren nach einem der Ansprüche 1 bis 4, dadurch gekennzeichnet, daß der Kontakt unter Verwendung eines Detergens in einer Menge von 0,1 bis 2,0 Gew.-%, bezogen auf das Gewicht der Teilchen mit einer Größe von mindestens 20 nm, erfolgt.

6. Verfahren nach Anspruch 5, dadurch gekennzeichnet, daß das Detergens in einer wäßrigen Lösung in einer Konzentration zwischen 0,5 und 1,0 Gew.-% vorhanden ist.

7. Verfahren nach einem der Ansprüche 1 bis 6, dadurch gekennzeichnet, daß (A) die antigene Masse, die Teilchen mit einer Größe von mindestens 20 nm enthält, zunächst über einen Zeitraum zwischen 0,1 und 4,0 Stunden, insbesondere zwischen 1,0 und 4,0 Stunden mit dem Detergens in Berührung gebracht wird und (B) dann die das Detergens enthaltende Antigen-Zusammensetzung mit einer wäßrigen Aldehydlösung in Berührung gebracht wird.

8. Verfahren nach Anspruch 7, dadurch gekennzeichnet, daß die Stufe (B) durchgeführt wird, indem die das Detergens enthaltende Antigen-Zusammensetzung mit dem Aldehyd bei einer Temperatur zwische 4 und 90°C, vorzugsweise zwischen 4 und 37°C über 1 bis 10 Tage in Berührung gehalten wird.

9. Verfahren nach einem der Ansprüche 1 bis 8, dadurch gekennzeichnet, daß das Detergens aus der Gruppe der Polyoxyäthylenderivate von Fettsäure-Partialestern von Sorbit-Anhydriden, z.B. einem Polyoxyäthylenderivat von Sorbitan-monooleat oder -monolaurat; Natriumdeoxycholat; synthetischen zwitterionischen Sulfobetain-Detergentien, wie N-Dodecyl-N,N-dimethyl-2-ammonio-1-äthansulfonat und verwandten Verbindungen; und Octyl-$\beta$-D-glucopyranosid ausgewählt ist.

10. Verfahren nach Anspruch 7 oder 8, dadurch gekennzeichnet, daß die nach der Stufe A erhaltene Masse steril filtriert und die nach der Stufe B erhaltene Masse einer Diafiltration unterzogen wird.

11. Verfahren nach Anspruch 10, dadurch gekennzeichnet, daß die nach der Diafiltration erhaltene Masse bis auf eine HBsAg-Konzentration von 10 bis 100 $\mu$g/ml verdünnt und mit Phosphat bis auf einen pH-Wert von 7,0 $\pm$ 0,5 gepuffert wird.

12. Verfahren nach Anspruch 11, dadurch gekennzeichnet, daß die nach der Pufferung erhaltene Masse steril filtriert, abgefüllt und lyophilisiert wird.

13. Verfahren nach einem der Ansprüche 1 bis 12, dadurch gekennzeichnet, daß die der Detergens-Behandlung unterzogene antigen Masse HBsAg-haltige Teilchen mit einer Teilchengröße von 16 bis 50 nm enthält.

14. Verfahren nach einem der Ansprüche 1 bis 13, dadurch gekennzeichnet, daß mindestens ein Teil der mit dem Detergens behandelten Teilchen e-Antigen enthält und/oder Dane-Teilchen oder -filamente darstellt.

15. Verfahren nach einem der Ansprüche 1 bis 14, dadurch gekennzeichnet, daß die subpartikulare Masse nach der Detergens-Behandlung eine Teilchengröße von weniger als 16 nm, vorzugsweise weniger als 5 nm und besonders bevorzugt zwischen 1 und 4 nm hat.

16. Verfahren nach einem der Ansprüche 1 bis 15, dadurch gekennzeichnet, daß die Antigen-Zusammensetzung praktisch frei von nachweisbarer DNS und/oder DNS-Polymerase und/oder Hepatitis-B-Kernantigen ist.

17. Verfahren nach einem der Ansprüche 1 bis 16, dadurch gekennzeichnet, daß die Antigen-Zusammensetzung praktisch frei von Antikörpern gegen Hepatitis-B-Oberflächenantigen (anti-HBs) und/oder von Antikörpern gegen e-Antigen (anti-HBe) ist.

18. Verfahren nach einem der Ansprüche 1 bis 17, dadurch gekennzeichnet, daß die subpartikulare Masse eine Dichte (im wäßrigen Medium) zwischen 1,21 und 1,24 g/cm$^3$ (bestimmt nach isopyknischen Banden in CsCl-Gradienten) hat.

19. Verfahren nach einem der Ansprüche 1 bis 18, dadurch gekennzeichnet, daß die subpartikulare Masse ein Molekulargewicht im bereich von 20 000 bis 1 000 000 Dalton hat.

20. Verfahren nach einem der Ansprüche 1 bis 19, dadurch gekennzeichnet, daß die subpartikulare Masse in der Antigen-Zusammensetzung in einer Menge von mindestens 0,001 Gew.-%, vorzugsweise in einer Menge zwischen 0,001 und 0,01 Gew.-% vorliegt.

21. Verfahren nach einem der Ansprüche 1 bis 20, zeichnet, daß die subpartikulare Masse in der Antigen-Zusammensetzung in einer solchen Menge vorhanden ist, daß bei einer Verabreichung an ein Lebewesen eine Antikörperreaktion erfolgt.

22. Verfahren nach einem der Ansprüche 1 bis 21, dadurch gekennzeichnet, daß die antigene Masse zusätzlich ein physiologisch verträgliches Medium enthält.

19

23. Verfahren nach einem der Ansprüche 1 bis 22, dadurch gekennzeichnet, daß die subpartikulare Masse eine im allgemeinen sphärische Morphologie hat.

ELECTRON MICROSCOPY OF FRACTIONS OF HBsAg/e PURIFIED BY
BATCHWISE ELUTION FROM HYDROXYLAPATITE AND RATE ZONAL CENTRIFUGATION

FIG. 1

FIG.2

FIG.3

Isopycnic main fraction Lot V

FIG.4

Isopycnic main fraction Lot VI

2

FIG.5

# FIG.6

## PURIFICATION SUMMARY

RUN NO: V

SUBTYPE: ay　　　　　　　　e: +

| STARTING MATERIAL | | |
|---|---|---|
| VOLUME | CEP TITER | TOTAL PROTEIN |
| 4,100 ml | 32-64 | 153,750 mg. |
| FOLD PURIFIC. | HBsAg YIELD | |
| 1.0 | 100% | |

FRACTIONAL　PEG PPTATION

| SECOND PEG PRECIPITATE | | |
|---|---|---|
| VOLUME | CEP TITER | TOTAL PROTEIN |
| 50.0 | 2560 | 1,720 mg |
| FOLD PURIFIC. | HBsAg YIELD | |
| 43.8-(87.6) | (49.0)-98.0% | |

pH 5.0　　PPTATION

SUPERNATE　　+ 1st WASH FLUID

| 3x WASHED PRECIPITATE | | |
|---|---|---|
| VOLUME | CEP TITER | TOT PROT |
| 20.0 ml | 2560 | 798.0 mg |
| FOLD PURIFIC. | HBsAg YIELD | |
| 77.4 | 39% | |

mg HBsAg: 51.2

| OH APATITE  0.02M-0.05 M ELUATE | | |
|---|---|---|
| VOLUME | CEP TITER | TOT. PROT. |
| 15.0ml | 2;560-5,120 | 74.1 |
| FOLD PURIFIC. | HBsAg YIELD | |
| 610 | 29.0 - 58.0 % | |

38.4 - 76.8

FINAL YIELD:

mg Serum Prot. 726.8

0 - 35.7

4

0 005 864

# FIG.7
SUMMARY OF PURIFICATION OF HBsAg/ay

| STARTING MATERIAL | Lot No. | V |
|---|---|---|
| | Subtype | ayw: eAg(+) |
| | Volume (ml) | 4,100.0 |
| | 1/Cep Titer | 32 |
| PEG/OH APATITE PRODUCT (A + B) | HBsAg Recovery(%) | 68–97 |
| | Mg HBsAg[1] | 89.6–128.0 |
| | Mg Total Protein[2] | 852.1 |
| | No. 30 $\mu$gm doses | 2,986–4,266 |
| | % particles >24 nm. | N.D. |
| (A + B) CsCl Flotation Isopycnic Banding Product | HBsAg Recovery(%) | 73.8 |
| | Mg HBsAg[3] | 96.9 |
| | Mg Total Protein[2] | 96.9 |
| | No. 30 $\mu$gm doses | 3,230 |
| | % Particle >24 nm | 5 – 10 |
| eAg in Final Product | | +(Faint) |

1  Based on 1000 CEP Units $\cong$ 1 mg/ml
2  Kjeldahl
3  O.D. 280nm; $E_{1\ cm}^{0.1\%} = 3.73$

5

# 0 005 864

# FIG.8

PURIFICATION SUMMARY

RUN NO: VI

SUBTYPE: adw          e:  +

| STARTING MATERIAL | | |
|---|---|---|
| VOLUME | CEP TITER | TOT.PROTEIN |
| 2,000 ml | 160-320 | 112,600.0 mg. |
| FOLD PURIFIC. | HBsAg YIELD | |
| 1x | 100% | |

FRACTIONAL   PEG PPTATION

| SECOND PEG PRECIPITATE | | |
|---|---|---|
| VOLUME | CEP TITER | TOT.PROTEIN |
| 100 ml | 4,000 | 6,000.0mg. |
| FOLD PURIF. | HBsAg YIELD | |
| 23.5x | >100% | |

pH 5.0   PPTATION

SUPERNATE   + 1st WASH FLUID

| 3x WASHED PRECIPITATE | | |
|---|---|---|
| VOLUME | CEP TITER | TOT.PROT |
| 25ml | 6,400 | 1,425.0 |
| FOLD PURIF. | HBsAg YIELD | |
| .39.5x | 50% | |

| OH APATITE  0.02 M-0.05M ELUATE | | |
|---|---|---|
| VOLUME | CEP TITER | TOT.PROTEIN |
| 9.0 | 12,800 | 170mg. |
| FOLD PURIFIC. | HBsAg YIELD | |
| 240x | 36 % | |

FINAL YIELD:

mg HBsAg:160.0          115.0

mg Serum Prot: 1,365.0          55.0

PARTICLES (EM):   ~25% > 24nm          5% >24nm

6

# FIG.9

## PURIFICATION SUMMARY

### LOT VI (adw)

| | Lot No. | VI |
|---|---|---|
| STARTING MATERIAL | Subtype | adw: eAg (+) |
| | Volume (ml) | 2,000.0 |
| | 1/CEP Titer | 160-320 |
| PEG/OH APATITE PRODUCT (A & B) | HBsAg Recovery (%) | 86% |
| | Mg. HBsAg[1] | 275.0 |
| | Mg. Total Protein[2] | 1,595.0 |
| | No. 30μgm doses [1] | 9,166 |
| | % particles > 24 nm | 10-25 |
| (A & B) CsCl Flotation Isopycnic Banding Product | HBsAg Recovery (%) | 78 |
| | Mg HBsAg[1] | 250 |
| | Mg. Total Protein[2] | 92.25 |
| | No 30 μgm doses [1] | 8,333[1] 3,075[2] |
| | % Particle >24 nm | 5-10 |
| eAg in Final Product | | (+) RIA |

[1] Based on 1000 CEP units = 1 mg/ml

[2] Kjeldahl

7